# EUROPEAN PATENT APPLICATION

(11) **EP 4 667 549 A2**
(43) Date of publication of application: **24.12.2025**
(21) Application number: 25179116.6
(22) Date of filing: 27.05.2025
(51) Int. Cl.: C09K 8/582, C12N 1/20, C12P 3/00

(54) **COMPOSITION AND METHOD FOR HYDROGEN PRODUCTION FROM HYDROCARBON-RICH RESERVOIRS**

(30) Priority: 27.05.2024 IN 202411040893
(71) Applicant: UAB GeoTransition Energy, 04105 Klaipeda (LT)
(72) Inventor: Haselton, Thomas Miles, LT00101 Palanga (LT); Pal, Mayur, LT-51368 Kaunas (LT); Lal, Banwari, 110003 New Delhi (IN); Baranauskas, Dr. Adomas, Vilnius (LT)
(74) Representative: Petraz, Gilberto Luigi

(57) **Abstract**

The present invention is a composition and method for hydrogen production from hydrocarbon-rich reservoirs. The composition comprises an anaerobic basal media (ABM) solution along with specific nutrients, vitamins, trace elements, surfactants, and an oxygen scavenger, formulated to optimize microbial growth and hydrogen production within the reservoir. The method of hydrogen extraction from hydrocarbon-rich reservoirs involves several key steps; a suitable hydrocarbon reservoir with an oil-containing zone and mobile water phase is selected, typically accessed through boreholes extending from the surface to the oil-bearing zone; a composition containing anaerobic basal media (ABM) and additional nutrients is injected into the reservoir through these boreholes, maintaining specific liquid temperature and pressure conditions between 60°C to less than 120°C and 150 to 300 bar, respectively.

## Description

### TECHNICAL FIELD OF THE INVENTION

The invention pertains to the field of hydrogen production. Specifically, it relates to a novel composition for hydrogen production and a method for extracting hydrogen gas efficiently and sustainably from hydrocarbon-rich reservoirs.

This application claims the benefit of Indian Application No. 202411040893 titled "Composition and method for hydrogen production from hydrocarbon-rich reservoirs" filed on May 27, 2024 which is incorporated herein by reference in its entirety.

### BACKGROUND OF THE INVENTION

Hydrogen is an important fuel and chemical process substrate of great economic importance. Hydrogen is increasingly recognized as a clean and efficient energy carrier with significant potential to reduce greenhouse gas emissions.

Hydrocarbon-rich deposits, such as depleted oil and gas reservoirs, represent vast reservoirs of organic material. Within these deposits, diverse microbial communities thrive in anaerobic conditions, capable of metabolizing hydrocarbons and producing hydrogen gas as a byproduct.

Hydrogen production from reservoirs involves leveraging microbial activity within hydrocarbon-rich deposits to generate hydrogen gas. This process utilizes indigenous microorganisms, which metabolize residual hydrocarbons present in the reservoir through anaerobic fermentation. By introducing solutions containing essential nutrients and vitamins into the reservoir, microbial growth and hydrogen production are stimulated. Controlled injection parameters and ongoing monitoring ensure optimal conditions for microbial activity, facilitating efficient extraction of hydrogen-rich fluids. The produced hydrogen gas can then be purified and utilized as a clean energy source across various applications, offering a renewable and environmentally friendly alternative to traditional fossil fuels.

Conventional methods of hydrogen production predominantly rely on fossil fuel-based processes such as steam methane reforming or coal gasification, which entail significant carbon emissions and environmental impact. These methods not only contribute to greenhouse gas emissions but also rely on finite and non-renewable resources, thereby posing challenges to long-term sustainability. Without targeted stimulation of microbial activity and optimization of growth conditions, the natural hydrogen production potential of indigenous microorganisms may not be fully realized using these methods.

Traditional microbiological processes face limitations in terms of efficiency and scalability. Existing compositions lack the precise formulation needed to optimize microbial growth and hydrogen production within hydrocarbon-rich deposits.

Furthermore, the lack of a standardized method for stimulating microbial activity and controlling operational parameters hampers the widespread adoption of microbiological hydrogen production.

WO2005113784 describes a method for enhancing microbial production of hydrogen from a hydrocarbon-rich deposit. The disclosure favors achieving this by stimulating the metabolic activities of indigenous microorganisms within the deposit, including by the introduction of exogenous (possibly genetically modified) organisms having metabolic capabilities of interest. These metabolic capabilities are not defined except insofar as their impact is to improve net hydrogen production, and contextually this seems to mean by inhibiting the consumption of hydrogen rather than by metabolization of hydrocarbons to hydrogen within the deposit. This document therefore fails to appreciate or to disclose the introduction into the deposit of further microorganisms which are non-native to the deposit and which themselves are capable of metabolizing hydrocarbons to molecular hydrogen and which serve to increase hydrogen production in the deposit by positively diversifying the microbiological abundance of microorganisms in the deposit.

Liu, J.-F. et al. (The diversity of hydrogen-producing microorganisms in a high-temperature oil reservoir and its potential role in promoting the in situ process, Applied Environmental Biotechnology, Vol. 2, pp. 25-34, 2016) examined hydrogen-producing microorganisms in the production water of an oil field as well as enrichment cultures Genes coding for [FeFe] hydrogenase using a clone library. The results showed that the [FeFe] hydrogenase genes in produced water are diverse and belong to *Bacteroidetes, Firmicutes, Spirochaetes* and uncultured. The results suggest that hydrogen-producing microorganisms in oil reservoirs can play a positive role in promoting the in situ bioprocess through hydrogen production once common nutrients are available.

The existing methodologies, as evidenced by conventional approaches and previous attempts, lack the requisite efficiency and scalability needed to fully leverage the potential of microbial activity within these reservoirs.

In view of the prior art examples and drawbacks, there is an evident need for an innovative solution that addresses these challenges and unlocks the full potential of hydrogen production from hydrocarbon-rich reservoirs. A novel composition for hydrogen production and method for extracting hydrogen gas from hydrocarbon-rich reservoirs has been developed.

### SUMMARY OF THE INVENTION

The present invention relates to a composition and method for hydrogen production from hydrocarbon-rich reservoirs, leveraging indigenous microbial activity and precise formulation of an anaerobic basal media (ABM) solution.

The composition enhances microbial population and enzymatic activity that is crucial for breaking down hydrocarbons into hydrogen gas. This approach maximizes hydrogen production without introducing additional microorganisms, offering a more efficient and sustainable method for utilizing microbial resources in hydrogen extraction from reservoirs.

In an embodiment of the present invention, the composition comprises an anaerobic basal media (ABM) solution along with specific nutrients, vitamins, trace elements, surfactants, and an oxygen scavenger, formulated to optimize microbial growth and hydrogen production within the reservoir.

In another embodiment of the present invention, the method of hydrogen extraction from hydrocarbon-rich reservoirs involves several key steps; a suitable hydrocarbon reservoir with an oil-containing zone and mobile water phase is selected, typically accessed through boreholes extending from the surface to the oil-bearing zone; a composition containing anaerobic basal media (ABM) and additional nutrients is injected into the reservoir through these boreholes, maintaining specific liquid temperature and pressure conditions between 60°C to less than 120°C and 150 to 300 bar, respectively.

In another embodiment of the present invention, following injection step, hydrogen-containing fluid is generated within the reservoir, hydrogen gas is extracted from the produced fluid, providing a clean and sustainable energy source.

In another embodiment of the present invention, the liquid injected in the composition may include a microorganism not naturally present in the hydrocarbon-rich reservoir; and/or a strain of microorganisms not naturally present in the hydrocarbon-rich reservoir; and/or a species of microorganisms not naturally present in the hydrocarbon-rich reservoir; and/or d. of a genus of microorganisms not naturally present in the hydrocarbon-rich reservoir.

It should be noted that while the present invention has been described with reference to fasteners, it is not limited to this particular type of manufactured object and can be adapted to inspect other types of objects as well. Additionally, various modifications and alterations to the system and method may be possible without departing from the scope of the invention.

### BRIEF DESCRIPTION OF THE DRAWINGS

The foregoing summary, as well as the following detailed description of various embodiments, is better understood when read in conjunction with the drawings provided herein. For the purposes of illustration, the drawings disclose subject matter which is not limited to the specific methods and instrumentalities disclosed. Further, the advantages and features of the present disclosure will better understood with reference to the following detailed description and claims taken in conjunction with the accompanying drawing, wherein like elements are identified with like symbols, and which:
**Figure 1** illustrates a graphical plot between OD (600nm) and time in hours in ABM + brine + oil sample;
**Figure 2** illustrates a graphical plot between Hydrogen production and time in hours in ABM + brine (10%) + oil (1%) sample;
**Figure 3** illustrates a graphical plot between OD (600nm) and time in hours in ABM + brine + oil sample with oxygen scavenger;
**Figure 4** illustrates a graphical plot between Hydrogen production and time in hours in ABM + brine (10%) + oil (1%) sample with an oxygen scavenger;
**Figure 5** illustrates a histogram depicting bacterial DNA present in oil reservoirs tested and expressed at the phylum level.; and
**Figure 6** illustrates a histogram depicting bacterial DNA present in oil reservoirs tested and expressed at class level;

The foregoing summary, as well as the following detailed description of the embodiments, will be better understood when read in conjunction with the attached drawings. For the purpose of illustration, there are shown in the drawings some embodiments, which may be preferable. It should be understood that the embodiments depicted are not limited to the precise details shown. Unless otherwise noted, the drawings are not to scale.

### DETAILED DESCRIPTION OF THE INVENTION

The following includes the preferred best mode of one embodiment of the present invention. It will be clear from this description of the invention that the invention is not limited to these illustrated embodiments but that the invention also includes a variety of modifications and embodiments thereto. Therefore, the present description should be seen as illustrative and not limiting. While the disclosed embodiment are susceptible to various modifications and alternative constructions, it should be understood, that there is no intention to limit the invention to the specific form disclosed, but, on the contrary, the invention is to cover all modifications, alternative constructions, and equivalents falling within the spirit and scope of the invention as defined in the claims.

In any embodiment described herein, the open-ended terms "comprising," "comprises," and the like (which are synonymous with "including," "having" and "characterized by") may be replaced by the respective partially closed phrases "consisting essentially of," consists essentially of," and the like or the respective closed phrases "consisting of," "consists of, the like.

As used herein, the singular forms "a," "an," and "the" designate both the singular and the plural, unless expressly stated to designate the singular only.

Further, the use of terms "first", "second", and "third", and the like, herein do not denote any order, quantity, or importance, but rather are used to distinguish one element from another.

The term "hydrocarbon-rich reservoir "refers to a subsurface geological formation containing substantial quantities of oil and/or natural gas.

The term "hydrocarbon" refers to a compound that contains only hydrogen and carbon atom.

The term "microorganisms" is intended to include bacteria and archaea organisms, their enzymes, and other products as well as relevant eukarya. It will be understood that bacteria and archaea are representative of microorganisms in general that can degrade petroleum and/or consume the resulting products under anoxic conditions.

The present invention relates to a composition and method for hydrogen production from hydrocarbon-rich reservoirs, leveraging indigenous microbial activity and precise formulation of anaerobic basal media (ABM) solution.

The composition for hydrogen production from hydrocarbon-rich reservoirs comprises an anaerobic basal media (ABM) solution supplemented with various components that include 1.0 - 100.0 g/L NH₄Cl; 0.33 g - 33.0 g/L MgCl₂.6H₂O ; 0.1 - 10.0 g/L CaCl₂.6H₂O; 0.33 - 33.0 g/L KCl ; 0.5 - 50.0 g/L KH₂PO₄ ; 4.0 - 400 g/L of yeast extract; 0.4 - 40 g/L of L-cysteine-HCl.H₂O; 0.01 - 1 g/L of at least one surfactant; and 1 ml of vitamin and trace element solution.

The trace element solution consists of micronutrients Nitrilotriacetic Acid, MgSO4 (Magnesium Sulfate), MnSO4 (Manganese Sulfate), NaCl (Sodium Chloride), FeSO4 (Iron Sulfate), CoCl2 (Cobalt Chloride), ZnSO4 (Zinc Sulfate), CuSO4 (Copper Sulfate), AlK (SO4)2 (Aluminum Potassium Sulfate), H3BO3 (Boric Acid) and Na2MoO4 (Sodium Molybdate).

These micronutrients act as cofactors and catalysts for essential metabolic reactions, ensuring the sustained activity of hydrogen-producing microorganisms.

Further, the amount of micronutrients in the composition including their concentration are 1.5 - 150.0 mg/L nitrilotriacetic acid; 3.0 - 300.0 mg/L MgSO₄; 0.5 - 50.0 mg/L MnSO₄; 1.0 - 100.0 mg/L NaCl; 0.1 - 10.0 mg/L FeSO₄; 0.1 - 10.0 mg/L CaCl₂; 0.1 - 10.0 mg/L CoCl₂; 0.1 - 10.0 mg/L ZnSO₄ ;0.01 - 1 mg/L CuSO₄ ; 0.01 - 1 mg/L AlK(SO4)₂; 0.01 - 1 g/L H₃BO₃ and 0.01 - 1 g/L Na₂MoO₄

The vitamin solution in the composition is selected from the group consisting of biotin, folic acid, pyridoxine hydrochloride, riboflavin, thiamine, nicotinic acid, pantothenic acid, vitamin B12, p-aminobenzoic acid and thioctic acid. These vitamins provide essential cofactors necessary for various enzymatic processes and metabolic pathways.

The composition may further comprise a carbohydrate source including but not limited to sugars (e.g., glucose, sucrose, fructose) and/or starches (e.g., corn starch, potato starch), with the flexibility to include alternative carbon sources such as industrial sugar or molasses amongst others.

Furthermore, the surfactant is selected from the group consisting of anionic surfactants, cationic surfactants, zwitterionic surfactants, non-ionic surfactants and combinations thereof. The surfactant employed in the present invention is Tween 80.

The yeast extract comprises various elements, including but not limited to amino acids, nitrogen sources.

Furthermore, there includes an oxygen scavenger in the composition to maintain anaerobic conditions, selected from the group consisting of L-cysteine-HCl-H₂O, sodium sulfite, erythorbate, diethylhydroxylamine (DEHA), hydroquinone, hydrazine, carbohydrazide, and methyl ethyl ketoxime (MEKO) amongst others. This compound effectively removes oxygen from the environment, preventing aerobic metabolism and facilitating the anaerobic conditions required for hydrogen generation.
The oxygen scavenger used in the composition is L-cysteine-HCl-H₂O.
The hydrocarbon-rich reservoir is preferably a liquid hydrocarbon-rich reservoir e.g., oil/bitumen/heavy oil.

The method of hydrogen extraction from a hydrocarbon-rich reservoir, comprises the steps of: using a hydrocarbon reservoir having an oil-containing zone with a mobile water phase, the hydrocarbon reservoir having at least one borehole extending from the surface of the hydrocarbon reservoir to an upper region or a lower region of the oil-bearing zone; injecting the composition comprising anaerobic basal media (ABM) and additional nutrients into at least one borehole of the reservoir at a liquid temperature in the range of 60 ° C to less than 120 ° C and pressure in the range of 150 to 300 bar; producing hydrogen-containing fluid from the hydrocarbon reservoir after opening the boreholes of the hydrocarbon reservoir that extend to the upper or lower region of the oil-containing zone of the reservoir; and extracting hydrogen gas from the hydrogen-containing fluid

The liquid used in the process may further contain at least one type of microorganism, optionally several types of microorganisms, which is/are suitable for converting hydrocarbons into hydrogen.

Furthermore, the liquid injected includes at least one type of microorganism, selected from the group consisting of thermophilic microorganisms, *Bacteroidetes, Firmicutes, Spirochaete, Clostridia and Syntrophus,* amongst others.

These microorganisms have proven to be particularly efficient in converting hydrocarbons into hydrogen.

The liquid is injected at a liquid temperature in the range from 60 - 120 °C, preferably a temperature from 70 °C to 110 °C.

The liquid used in the process can be injected at a pressure in the range of 120 to 350 bar, preferably 150 to 300 bar. This pressure range represents an optimum because injection must not occur above fracking pressure and injection below reservoir pressure is not possible.

In the method, a pH regulator is implemented within the reservoir to regulate the pH of the hydrogen-producing microorganisms in the range of 6.0 - 8.0.

The pH regulator may optionally also serve as a nutrient - for example, phosphate can act as both a nutrient and as a buffering agent ensuring a balanced microbial ecosystem conducive to sustained hydrogen production.

The upper region of the oil-containing zone of the hydrocarbon reservoir can be a region of the oil-containing zone which extends over a certain length from an uppermost point of the oil-containing zone in a direction along the force of gravity to the lowest point of the oil-containing zone, wherein the lowest point of the oil-containing zone represents the end of the hydrocarbon reservoir or a beginning of a water zone of the hydrocarbon reservoir,

The lower region of the oil-containing zone of the hydrocarbon reservoir can be a region of the oil-containing zone which extends over a certain length from the lowest point of the oil-containing zone in a direction counter to gravity to the highest point of the oil-containing zone, wherein the lowest point of the oil-containing zone represents the end of the hydrocarbon reservoir or a beginning of a water zone of the hydrocarbon reservoir.

The enzyme activity enhancement and microbial population boosting are achieved through the regulation of temperature, pH, nutrient availability, and the addition of suitable growth promoters.

Additionally, the concentration of the ABM solution injected into the reservoir is optimized based on the characteristics of the indigenous microbial population and the desired rate of hydrogen production.

### EXAMPLES

### METHODOLOGY

### A. Preparation of Anaerobic basal media composition or recipe - In this experiment the refinement of anaerobic basal media (ABM) was conducted to maximize bacterial growth and subsequent hydrogen gas yield under different temperature conditions. Materials and Methods

In the experiment, ABM + oil + brine mixtures were dispensed into 100 mL bottles, leaving a working volume of 30 mL. Bottles were purged with pure N2 gas to create an anaerobic environment and sealed with rubber septa and aluminium crimp caps. Furthermore, the experiments were conducted at both 60°C and 80°C, with similar growth observed at both temperatures.
a. Cultivation Phase:
   - Brine samples (10% v/v) were incorporated into ABM solutions at 60°C or 80°C.
   - ABM formulations included essential nutrients such as Nitrogen and Phosphorus sources, trace elements, and vitamins to support bacterial growth and enzymatic activity.
   - Bacterial growth kinetics were monitored over 200 hours, with peak concentrations determined by measuring solution turbidity at OD600 nm.
   - Periodic sampling involved drying aliquots and microscopic examination to visualize bacterial colonies.
b. Hydrogen Production Phase:
   - Peak bacterial cultures from the cultivation phase were inoculated into fresh ABM supplemented with oil (3.3% v/v) in specialized growth bottles.
   - The objective was to stimulate further bacterial growth and quantify hydrogen gas production using gas chromatography.
   - Gas analysis included sampling 2.0 mL of gas from the headspace of each bottle, followed by gas chromatography analysis using Agilent 7890B GC system.

### Observations and findings

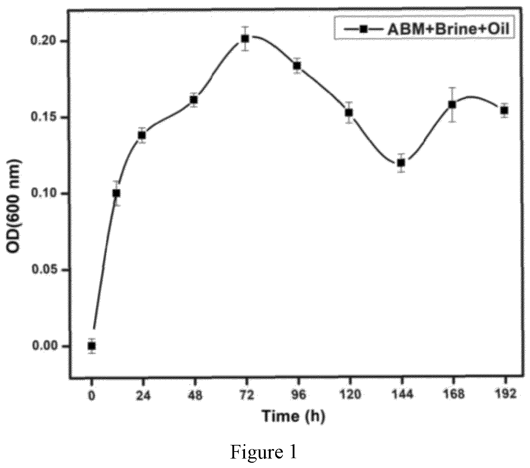

The graph of Figure 1 plotted between Optical density measurements of ABM + brine + oil sample and time (hours) shows that bacteria reach their highest peak growth at 72 hours at an optical density of 0.20. Figure 1 illustrates the utilization of the optimized ABM formulation of ABM + brine + oil sample to promote robust bacterial growth.

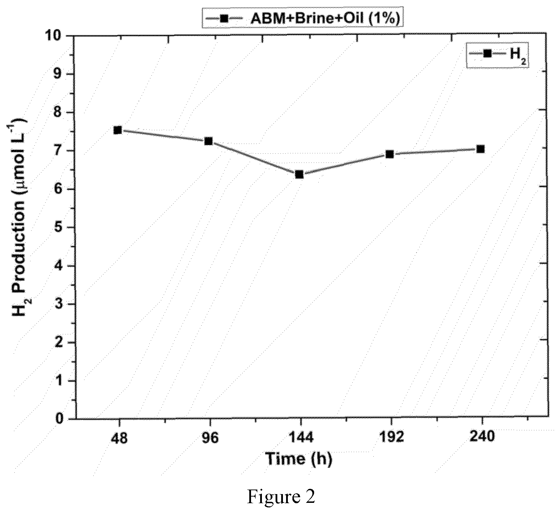

Figure 2 demonstrates the effectiveness of the ABM composition in facilitating hydrogen gas production throughout the 240-hour timeframe in ABM + brine (10%) + oil (1%) sample. The graph of Figure 2 plotted between Hydrogen production and time (hours) shows that about 7.5 bacterial hydrogen is produced at 48 hours

The ABM composition exhibits remarkable potential in enhancing anaerobic bacterial growth and subsequent hydrogen gas production. These findings highlight the importance of optimizing ABM formulations for biotechnological applications, offering insights into sustainable pathways for microbial cultivation and gas generation.

### B. Preparation of Anaerobic basal media composition or recipe with oxygen scavenger

This experiment determines the efficacy of a simplified ABM recipe supplemented with an oxygen scavenger, L-cysteine-HCl-H2O, to maintain anaerobic conditions while promoting efficient bacterial growth and hydrogen production.

### Materials and Methods:

a. Cultivation Phase:
   ABM Composition:
   a NH4Cl: 1 g/L
   b KH2PO4: 0.5 g/L
   c Yeast extract: 4.0 g/L
   d L-cysteine-HCl-H2O (oxygen scavenger): 0.4 g/L
      - Brine (10% v/v) and oil (2% v/v) were added to the ABM solution.
      - Bacterial growth kinetics were monitored by measuring the optical density at 600 nm (OD600) over a 72-hour period.
      - Sampling involved periodic measurements to determine peak bacterial growth.
b. Hydrogen Production Phase:
   - ABM composition remained consistent with the cultivation phase.
   - Sucrose was supplemented to observe hydrogen production rescue after the plateau.
   - Hydrogen gas production was monitored over a 288-hour timeframe.
   - Samples were periodically analyzed to quantify hydrogen gas content using gas chromatography.

### Observations and Findings:

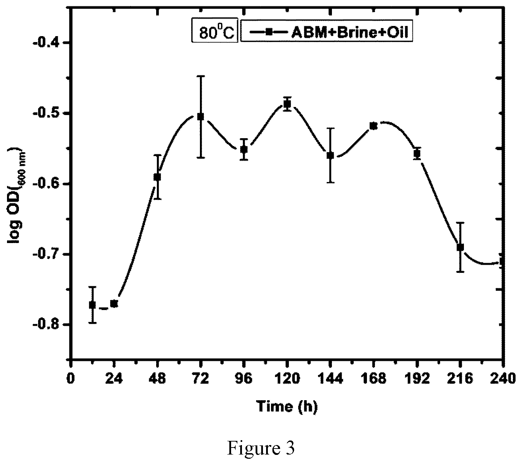

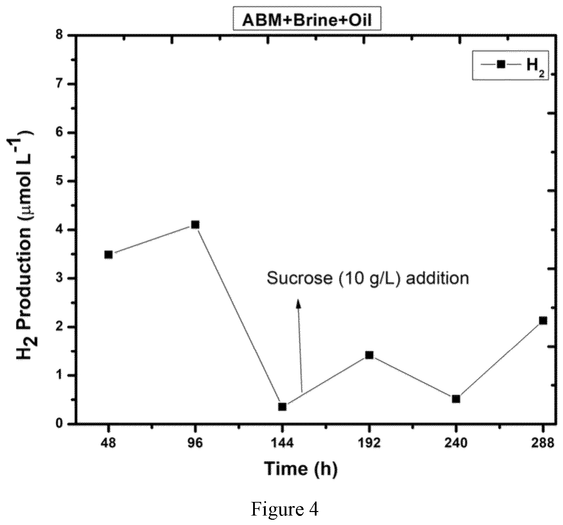

The plot of Figure 3 depicted a steady increase in bacterial growth, with peak growth observed at 72 hours. The presence of the oxygen scavenger ensures anaerobic conditions, facilitating efficient bacterial proliferation. The plot of Figure 4 mentions that the sustained hydrogen production indicates the effectiveness of the oxygen scavenger in maintaining anaerobic conditions. Furthermore, sucrose supplementation demonstrates the ability to overcome production plateaus, leading to enhanced hydrogen yield.

The simplified ABM recipe, supplemented with an oxygen scavenger, proves effective in supporting robust bacterial growth and sustained hydrogen gas production.

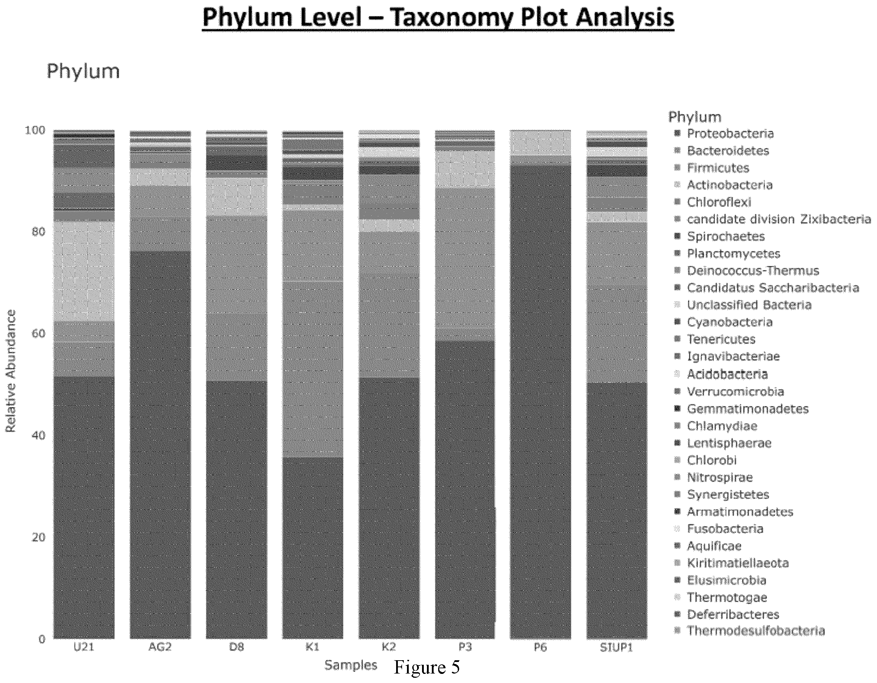

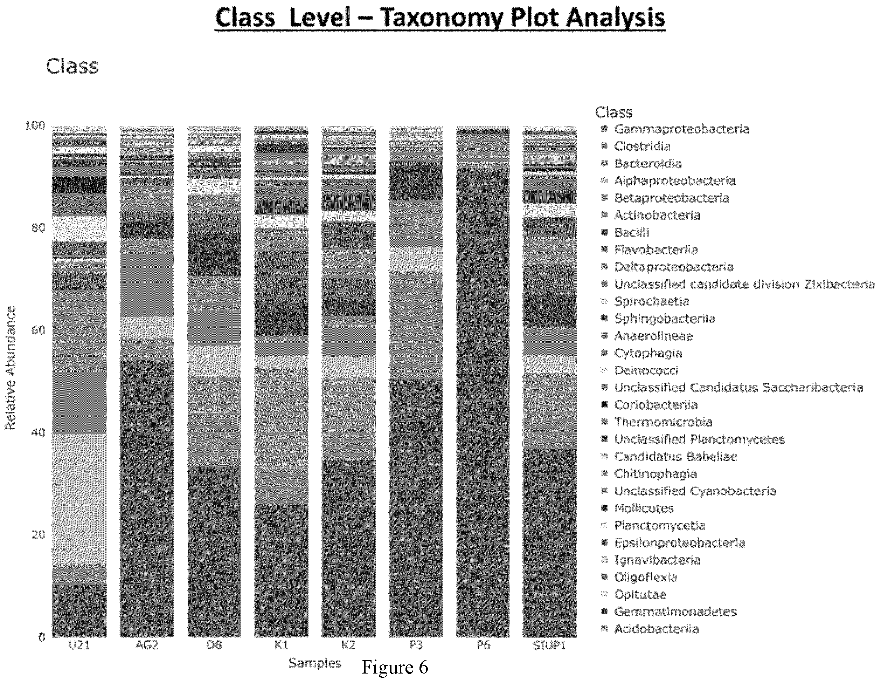

DNA present in brine collected from the oil reservoirs was PCR amplified, sequenced and compared to known bioinformatics DNA libraries of bacteria at phylum and class levels. The DNA found in reservoir brine shows hits with Proteobacteria, Firmicutes, Bacteroidetes, and Spirochaetes bacterial sequences at phylum level (Figure 5). These are known producers of hydrogen, for example, Clostridia and Bacilli from the firmicutes phylum shown in Class level (Figure 6).

### Hydrogen Extraction Methodology from Hydrocarbon-Rich Reservoirs

In the process of extracting hydrogen from hydrocarbon-rich reservoirs, a meticulous methodology is employed to ensure accurate analysis and understanding of the gas composition. Gas samples are carefully harvested from oil reservoirs and stored in sterilized containers to prevent contamination. Subsequently, a comprehensive analytical approach is undertaken, utilizing advanced techniques such as isotope ratio mass spectrometry (IRMS) and gas chromatography (GC) to elucidate both the isotopic and chemical composition of the collected gases.

### Gas Composition Analysis (Table 1):

**Table 1**

| Compound | In bulk sample [% vol] | In sample (without air contamination) [% vol] |
|---|---|---|
| C₁ | 38.13007 | 38.28366 |
| C₂ | 11.07247 | 11.11707 |
| C₃ | 14.95245 | 15.01268 |
| i-C₄ | 2.61052 | 2.62103 |
| n-C₄ | 8.68477 | 8.71975 |
| neo-C₅ | 0.02564 | 0.02575 |
| i-C₅ | 2.83800 | 2.84943 |
| n-C₅ | 4.18616 | 4.20302 |
| Σc₆ | 2.81263 | 2.82396 |
| ΣC₇ | 1.84730 | 1.85474 |
| ΣC₈ | 0.21590 | 0.21677 |
| ΣC₉ | 0.00518 | 0.00520 |
| ΣC₁₀ | 0.00000 | 0.00000 |
| CO₂ | 4.74865 | 4.76763 |
| N₂ | 7.31892 | 7.03107 |
| O₂ | 0.08496 | 0.00000 |
| CO | 0.00000 | 0.00000 |
| He | 0.18800 | 0.18876 |
| H₂ | 0.27833 | 0.27945 |
| H₂S | 0.00000 | 0.00000 |
| Methyl mercaptan | 0.00002 | 0.00002 |
| Ethyl mercaptan | 0.00001 | 0.00001 |
| Dimethyl sulfide | 0.00000 | 0.00000 |
| i-propyl mercaptan | <0.00001 | <0.00001 |
| n-propyl mercaptan | <0.00001 | <0.00001 |
| i-butyl mercaptan | <0.00001 | <0.00001 |
| n-butyl mercaptan | <0.00001 | <0.00001 |

Gas chromatography, a powerful analytical tool, is utilized to discern the precise chemical composition of the gas samples obtained from the well. This detailed analysis reveals the presence of significant amounts of hydrogen gas, among other constituents. The gas chromatographic data offers insights into the relative abundance of various components within the gas phase, providing crucial information for further interpretation.

### Isotopic Analysis of Nearby Oil Reservoirs in Western Lithuania (Table 2):

In parallel with the chemical composition analysis, isotopic analysis is conducted to probe the origin of the hydrogen gas present in the gas phases of nearby oil reservoirs, particularly those situated in Western Lithuania. Isotope ratio mass spectrometry (IRMS) is employed to determine the isotopic composition of hydrogen, specifically focusing on the δD-H2 values, which serve as indicators of the hydrogen's source. The observed high δD-H2 values suggest a biogenic origin for the hydrogen, indicating that it likely originated from biological processes associated with organic matter decomposition.

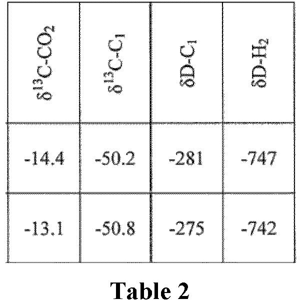

Moreover, the ratio of C1/C2 carbons, derived from the gas chromatographic data (Table 1), further supports the inference of a biogenic source for the hydrogen. The elevated ratio of C1/C2 carbons is consistent with the composition typically associated with biogenic methane, reinforcing the notion that hydrogen gas is a byproduct of microbial activity within the reservoir.

While the preferred embodiments have been shown and described, it will be understood that there is no intent to limit the invention by such disclosure, but rather, is intended to cover all modifications and alternate constructions falling within the spirit and scope of the invention.

## Claims

1. A composition for hydrogen production from a hydrocarbon-rich reservoir, comprising anaerobic basal media (ABM) solution, wherein said composition further consists of;
a. 1.0 - 100.0 g/L NH₄Cl;
b. 0.33 g - 33.0 g/L MgCl₂.6H₂O;
c. 0.1 - 10.0 g/L CaCl₂.6H₂O;
d. 0.33 - 33.0 g/L KCl;
e. 0.5 - 50.0 g/L KH₂PO₄;
f. 4.0 - 400 g/L of yeast extract;
g. 0.4 - 40 g/L of L-cysteine-HCl.H₂0;
h. 0.01 - 1 g/L of at least one surfactant; and
i. 1 ml/L of vitamin and trace element solution.

2. The composition of Claim 1, wherein the trace element solution consists of micronutrients including but not limited to Nitrilotriacetic Acid, MgSO4 (Magnesium Sulfate), MnSO4 (Manganese Sulfate), NaCl (Sodium Chloride), FeSO4 (Iron Sulfate), CoCl2 (Cobalt Chloride), ZnSO4 (Zinc Sulfate), CuSO4 (Copper Sulfate), AlK (SO4)2 (Aluminum Potassium Sulfate), H3BO3 (Boric Acid) and Na2MoO4 (Sodium Molybdate).

3. The composition of Claim 2, wherein the final concentration of the trace elements in the solution includes;
a. 1.5 - 150.0 mg/L nitrilotriacetic acid;
b. 3.0 - 300.0 mg/L MgSO₄;
c. 0.5 - 50.0 mg/L MnSO₄;
d. 1.0 - 100.0 mg/L NaCl;
e. 0.1 - 10.0 mg/L FeSO₄;
f. 0.1 - 10.0 mg/L CaCl₂;
g. 0.1 - 10.0 mg/L CoCl₂;
h. 0.1 - 10.0 mg/L ZnSO₄;
i. 0.01 - 1 mg/L CuSO₄;
j. 0.01 - 1 mg/L AlK(SO4)₂;
k. 0.01 - 1 g/L H₃BO₃; and
l. 0.01 - 1 g/L Na₂MoO₄.

4. The composition of Claim 1, wherein the vitamin solution is selected from the group consisting of biotin, folic acid, pyridoxine hydrochloride, riboflavin, thiamine, nicotinic acid, pantothenic acid, vitamin b12, p-aminobenzoic acid and thioctic acid.

5. The composition of Claim 4, wherein the final concentration of the vitamins and nutrients related to the vitamin solution includes;
a. 2.0 - 200 µg/L biotin;
b. 2.0 - 200 µg/L folic acid;
c. 2.0 - 200 µg/L pyridoxine hydrochloride;
d. 10.0 - 1 µg/L riboflavin;
e. 5.0 - 500 µg/L thiamine;
f. 5.0 - 500 µg/L nicotinic acid;
g. 5.0 - 500 µg/L pantothenic acid;
h. 0.1 - 100 µg/L vitamin B12;
i. 5.0 - 500 µg/L p-aminobenzoic acid and
j. 5.0 - 500 µg/L thioctic acid.

6. The composition of Claim 1, wherein the composition may comprise a carbohydrate source including but not limited to sugars (e.g., glucose, sucrose, fructose) and/or starches (e.g., corn starch, potato starch), with the flexibility to include alternative carbon sources such as industrial sugar or molasses amongst others.

7. The composition of Claim 1, wherein the surfactant is selected from the group consisting of anionic surfactants, cationic surfactants, zwitterionic surfactants, non-ionic surfactants and combinations thereof.

8. The composition of Claim 1 or 7, wherein the surfactant is preferably Tween 80.

9. The composition of Claim 1, wherein the yeast extract comprises various elements, including but not limited to amino acids, and nitrogen sources.

10. The composition of Claim 1, further comprising an oxygen scavenger to maintain anaerobic conditions, wherein said oxygen scavenger is selected from the group consisting of L-cysteine-HCl-H2O, sodium sulfite, erythorbate, diethylhydroxylamine (DEHA), hydroquinone, hydrazine, carbohydrazide, and methyl ethyl ketoxime (MEKO) amongst others.

11. The composition of Claim 1 or 10, wherein the oxygen scavenger used in the composition is L-cysteine-HCl-H₂O.

12. The composition of Claim 1, wherein the anaerobic basal media (ABM) solution is designed to maintain a stable pH range conducive to the growth and metabolic activity of thermophilic microorganisms.

13. The composition of Claim 12, wherein at least one nutrient is selected to promote the growth of the microorganism(s) in the reservoir.

14. A method of hydrogen extraction from a hydrocarbon-rich reservoir, comprising the steps of:
a) using a hydrocarbon reservoir having an oil-containing zone with a mobile water phase, the hydrocarbon reservoir having at least one borehole extending from the surface of the hydrocarbon reservoir to an upper region or a lower region of the oil-bearing zone;
b) injecting the composition comprising anaerobic basal media (ABM) and additional nutrients into at least one borehole of the reservoir at a liquid temperature in the range of 60 ° C to less than 120 ° C and pressure in the range of 150 to 300 bar;
c) producing hydrogen-containing fluid from the hydrocarbon reservoir after opening the boreholes of the hydrocarbon reservoir that extend to the upper or lower region of the oil-containing zone of the reservoir; and
d) extracting hydrogen gas from the hydrogen-containing fluid

15. The method as claimed in Claim 14, wherein the liquid is injected at a liquid temperature in the range from 60 - 120 °C, preferably a temperature from 70 °C to 110 °C.

16. The method as claimed in Claim 14, wherein the liquid used in the method can be injected at a pressure in the range of 120 to 350 bar, preferably 150 to 300 bar.

17. The method as claimed in any one of the preceding claims, wherein a pH regulator is implemented within the reservoir to regulate the pH of the hydrogen-producing microorganisms in the range of 6.0 - 8.0.

18. The method as claimed in any one of the preceding claims, wherein the liquid injected includes at least one type of microorganism, selected from the group consisting of thermophilic microorganisms, *Proteobacteria, Firmicutes, Bacteroidetes and Spirochaetes* amongst others.

19. The method of Claim 14, wherein enzyme activity enhancement and microbial population boosting are achieved through the regulation of temperature, pH, nutrient availability, and the addition of suitable growth promoters.

20. The method of Claim 14, wherein the concentration of the ABM solution injected into the reservoir is optimized based on the characteristics of the indigenous microbial population and the desired rate of hydrogen production.

21. The method according to any one of claims 14 to 19 wherein the hydrocarbon-rich reservoir is a liquid hydrocarbon-rich reservoir including but not limited to oil/bitumen/heavy oil.
